# FASCICULE DE BREVET EUROPEEN

(11) **EP 0 680 315 B1**
(45) Date de publication et mention de la délivrance du brevet: **17.01.2001**
(21) Numéro de dépôt: 94931633.5
(22) Date de dépôt: 26.10.1994
(51) Int. Cl.: A61K 9/00

(54) **PROCEDE DE PREPARATION D'UN AEROSOL VIRAL**
VERFAHREN ZUR HERSTELLUNG EINES VIRUS AEROSOLS
METHOD FOR PREPARING A VIRAL AEROSOL

(30) Priorité: 26.10.1993 FR 9312743
(43) Date de publication de la demande: 08.11.1995
(73) Titulaire: TRANSGENE S.A., 67082 Strasbourg Cédex (FR)
(72) Inventeur: SENE, Claude, F-67190 Mutzig (FR); LAMY, Didier, F-67000 Strasbourg (FR)
(74) Mandataire: Warcoin, Jacques
(86) Numéro de dépôt international: FR9401245
(87) Numéro de publication internationale: WO9511664

(56) Documents cités:
- FR-A- 2 423 217
- US-A- 7 769 623

## Description

La présente invention a pour objet un procédé de préparation d'un aérosol comprenant un virus ainsi que l'utilisation d'un tel aérosol pour la préparation d'un médicament.

La formulation aérosol de diverses substances, notamment médicamenteuses, est connue depuis longtemps. La stabilité et la vitesse de chute négligeable constituent les caractéristiques essentielles d'un aérosol. C'est pourquoi, d'une part, afin d'éviter que les particules ne s'aggrègent entre elles ou présentent une vitesse de chute trop élevée, propriétés incompatibles avec une bonne efficacité, le dosage doit répondre à un certain nombre de contraintes spécifiques, tenant notamment à différents paramètres d'environnement tels que le degré d'humidité et la température, et, d'autre part, la formulation doit tenir compte d'un certain nombre de difficultés liées principalement à l'efficacité de la défense du tractus respiratoire contre les contaminants (mécanismes de clairance), qui tend à dégrader rapidement les principes actifs inhalés.

En particulier, dans le cas où le principe actif est constitué par des virus, l'intégrité de la particule virale est nécessaire pour l'infection et la pénétration dans les cellules de l'épithélium pulmonaire, ce qui impose des conditions d'obtention de l'aérosol très spécifiques.

D'une manière générale, lors d'une aérolisation, un virus peut être inactivé en raison de trois causes majeures. Tout d'abord, la perte du pouvoir infectieux peut résulter des dommages subis par le virus pendant le processus de nébulisation (pulvérisation dans les voies respiratoires), il peut également être inactivé dans l'aérosol par déshydratation, enfin, il peut être dégradé dans l'environnement potentiellement hostile que constitue le mucus qui recouvre l'ensemble du tractus respiratoire et qui contient un grand nombre d'enzymes (protéolytiques et autres).

On a maintenant mis en évidence que l'inaction virale pouvait être limitée et maintenue dans des limites raisonnables en appliquant pour l'obtention de l'aérosol le procédé selon l'invention mettant en oeuvre des conditions particulières de nébulisation et de conditionnement. Ce procédé permet, en outre, de délivrer efficacement et en quantité appropriée le virus dans le poumon, notamment au niveau de la voie trachéo-bronchique.

C'est pourquoi la présent invention a pour objet un procédé d'obtention d'un aérosol viral caractérisé en ce que l'on prépare une suspension virale diluée correspondant à la dilution d'une suspension virale contenant de 10⁴ à 10¹³ unités formant des plages (ufp) d'un virus dans une solution aqueuse contenant au moins de 6 à 12 g/l d'un sel d'un cation monovalent ou de 50 à 100 g/l d'un hexose.

Le procédé selon la présente invention est applicable à un très grand nombre de virus administrable par voie aérosol, notamment les virus choisis dans le groupe constitué par les poxvirus, rétrovirus, virus de l'herpès, virus associé à l'adénovirus, rhinovirus, virus de l'influenza et adénovirus.

Dans le cadre de la présente invention, le terme « virus » désigne aussi bien un virus naturel , tel que trouvé dans la nature, qu'un virus modifié, dont le génome comporte des modifications par rapport à celui du virus parental dont il est issu. Il peut s'agir d'un virus atténué qui a perdu tout ou partie de son pouvoir pathogène par rapport au virus naturel dont il dérive. Son génome est modifié *in vivo* au cours des passages successifs en culture cellulaire ou dans un organisme vivant.

Le terme "virus" peut faire également référence à un virus recombinant, dont le génome est modifié *in vitro* par les techniques du génie génétique. La modification peut, par exemple, permettre d'inactiver au moins un gène essentiel à la réplication virale (rendant le virus défectif pour la réplication) et/ou d'insérer un fragment d'ADN codant pour une protéine hétérologue (normalement non codée par le virus naturel). L'insertion a lieu dans une région appropriée du génome viral, de manière à permettre l'expression du fragment d'ADN hétérologue dans une cellule hôte. Une cellule hôte est constituée par toute cellule eucaryote infectable par ledit virus, avantageusement une cellule humaine et de préférence une cellule épithéliale de la voie trachéo-bronchique.

Aux fins de la présente invention, le fragment d'ADN hétérologue peut être issu d'un organisme eucaryote ou d'un virus autre que celui dans lequel il est inséré. Il peut être isolé par toute technique conventionnelle dans le domaine de l'art, par exemple par clonage, PCR (Polymerase Chain Reaction) ou synthèse chimique. Il peut s'agir d'un fragment d'ADN de type génomique (comportant tout ou partie de l'ensemble des introns du gène naturel), de type ADN complémentaire (ADNc ; dépourvu d'introns) ou de type minigène, c'est-à-dire mixte (comportant au moins tout ou partie d'un intron).

Conformément aux buts poursuivis par la présente invention, le fragment d'ADN hétérologue peut être placé sous le contrôle des éléments nécessaires à son expression. Par "éléments nécessaires", on désigne l'ensemble des éléments permettant la transcription dudit fragment d'ADN en ARN messager (ARNm) ou la traduction de l'ARNm en protéine.

Le fragment d'ADN hétérologue peut coder pour une protéine (i) intracellulaire, (ii) membranaire présente à la surface de la cellule hôte ou (iii) sécrétée dans le milieu extérieur. Il peut donc comporter une séquence signal permettant la sécrétion de la protéine vers la membrane ou hors de la cellule hôte.

De nombreux virus recombinants pouvant bénéficier d'une administration par voie aérosol sont décrits dans l'art antérieur. Leurs construction et propagation sont à la portée de l'homme du métier. A titre d'exemples, on peut citer l'Ad-α-1AT (Rosenfeld et al., 1991, Science, 252, 431-434), dans le génome duquel est inséré le gène humain codant pour l'α1 antitrypsine (α1AT) et l'Ad-CFTR (Rosenfeld et al., 1992, Cell, 68, 143-155), dans le génome duquel est inséré le gène humain codant pour la protéine CFTR (pour Cystic Fibrosis Transmembrane Conductance Regulator, en anglais).

Le procédé selon la présente invention implique la préparation d'une suspension virale diluée. Cette suspension virale diluée correspond à la dilution d'une suspension virale contenant de 10⁴ à 10¹³ upf de virus dans une solution aqueuse contenant au moins de 6 à 12 g/l d'un sel de cation monovalent ou de 50 à 100 g/l d'un hexose.

Dans cette étape, la suspension à diluer est généralement formée de virus placés dans un milieu tamponné contenant éventuellement un cation divalent, tel que le magnésium, le calcium ou le manganèse. Ce type de suspension étant également utilisable pour la conservation. Pour une conservation sous forme congelée, il convient de supplémenter la suspension avec un agent stabilisant tel que le glycérol, à une concentration d'au moins 10 % ou le saccharose à une concentration d'environ 1 M.

La suspension de particules virales à diluer peut, éventuellement, comprendre d'autres substances, notamment l'albumine sérique humaine (HSA), l'urée, le glutamate de sodium, la glycine et l'inositol.

Selon le procédé de la présente invention, il est nécessaire que la suspension virale comprenne de 10⁴ à 10¹³, avantageusement de 10⁶ à 10¹² et de préférence de 10⁸ à 10¹¹ upf d'un virus, l'activité de cette suspension pourra notamment dépendre du virus utilisé.

Cette suspension à diluer est ensuite diluée avec la solution aqueuse selon un rapport en volume suspension /solution aqueuse de 1/5 à 1/20, avantageusement de 1/10 à 1/20, de préférence de 1/12 à 1/18 et de manière tout à fait préférée environ 1/16.

La solution aqueuse comprend de préférence de 6 à 12 g/l d'un sel d'un cation monovalent, de préférence un sel de sodium ou un sel de potassium et de manière tout à fait préférée le chlorure de potassium, le lactate de sodium et/ou le chlorure de sodium. On préfère une concentration en sel d'un cation monovalent de 6 à 10 g/l et de manière tout à fait préférée d'environ 9 g/l.

Lorsque la solution aqueuse comprend de 50 à 100 g/l d'un hexose et de préférence environ 50 g/l, il s'agit notamment de glucose et de mannose.

En outre, la solution aqueuse peut comprendre d'autres composés, tels qu'un sel de calcium, par exemple du chlorure de calcium.

La suspension ainsi diluée permet de générer un aérosol dont la stabilité particulière du tractus respiratoire est suffisante pour permettre le passage du virus. Bien entendu, il est possible d'obtenir une suspension virale correspondante par une autre séquence de manipulation.

Le procédé selon l'invention est tout particulièrement adapté à la préparation d'un aérosol destiné à des fins thérapeutiques, pour délivrer une quantité optimale de particules virales au niveau des voies trachéobronchiques du tractus respiratoire.

D'une manière générale, chez l'homme, le tractus respiratoire est composé de trois régions disctinctes :
- la partie supérieure qui s'étend du nez jusqu'au sommet de la trachée ;
- Les voies trachéo-bronchiques qui vont du haut de la trachée jusqu'aux bronchioles terminales ; et
- la région alvéolaire qui s'étend des bronchioles aux sacs alvéolaires.

Parmi les maladies pouvant être traitées en mettant en oeuvre le procédé selon l'invention, on peut citer les maladies pulmonaires touchant les voies trachéo-bronchiques et notamment la mucoviscidose, l'emphysème pulmonaire, l'asthme et le cancer du poumon.

Dans ce contexte, les virus selon l'invention sont de préférence des virus recombinants dont le génome comprend un fragment d'ADN codant pour une protéine hétérologue capable notamment d'inhiber, ralentir la progression d'une maladie pulmonaire ou empêcher son établissement. Parmi les protéines hétérologues, on peut citer celles qui sont capables :
de participer directement ou indirectement au transport des ions à travers les membranes cellulaires, et plus spécifiquement au transport des ions chlorure (CI⁻) ou sodium (Na⁺), comme la protéine CFTR (Riordan et al., Science, 245, 1066-1073) ;
- de réduire l'activité des protéases présentes dans les poumons, notamment dans des conditions inflammatoires, comme l'α1AT native (Long et al., 1984, Biochemistry, 23, 4828 - 4837) ou modifiée (Jalllat et al., 1986, Protein Engineering, 1, 29-35) ; et
- d'inhiber la croissance des cellules tumorales en renforçant l'immunité cellulaire, comme les interleukines (IL), les interférons (IFN) ou les facteurs nécrosant les tumeurs (TNF) et en ayant une activité suppressive de tumeur comme la protéine p53 (Baker et al., 1989, Science, 244, 217 - 221) ou Rb (Friend et al., 1986, Nature, 323, 643646).

Cette liste n'est pas limitative des fragments. Des fragments d'ADN codant pour d'autres protéines décrites dans la littérature pour leur effet anti-tumoral ou leur effet d'inhibiteur à l'égard de la destruction des tissus pulmonaires peuvent être utilisés.

Ainsi, l'invention concerne également l'utilisation d'un aérosol viral susceptible d'être obtenu par le procédé ci-dessus décrit, pour la préparation d'un médicament administré par nébulisation de ladite suspension virale diluée avec une pression de gaz de 0,5 à 3,5 bars ou une fréquence ultrasonore de 2 à 5 MHz.

La nébulisation de la suspension diluée peut être réalisée par une pression de gaz ou par des ultrasons. Les conditions de cette nébulisation constituent également des paramètres critiques de mise en oeuvre du procédé. Selon un mode préféré de mise en oeuvre du procédé selon la présente invention, la suspension diluée est soumise à une pression de gaz de 0,5 à 3,5 bars, et de manière tout à fait préférée de 2 à 3,5 bars. D'une manière alternative, elle peut également être soumise à une fréquence ultrasonore de 5 à 5 MHz et, de préférence, environ 2 à 3 MHz. La pression gazeuse ainsi que la fréquence ultrasonore peuvent être appliquées au moyen d'un nébuliseur.

D'une manière générale, un nébuliseur est un appareil permettant l'administration des aérosols. Les nébuliseurs peuvent être de type quelconque et leurs structures sont connues de l'homme de métier et ces appareils sont disponibles commercialement.

Lorsqu'il s'agit d'appliquer une pression gazeuse, on met en oeuvre, de préférence, un nébuliseur de type pneumatique relié soit à une source de gaz comprimé, comme l'air ou l'oxygène médical, soit à une pompe pneumatique. S'agissant de nébuliser une suspension virale diluée avec une fréquence ultrasonore, on préfère utiliser un nébuliser de type ultrasonique, lequel est pourvu d'un quartz vibrant à haute fréquence.

L'inhalation peut avoir lieu en dose unique ou répétée, une ou plusieurs fois après un certain délai d'intervalle.

L'invention est plus complètement décrite à l'aide des exemples suivants :

### EXEMPLE 1 : Préparation d'un aérosol à partir d'une suspension virale conservée en présence de glycérol 10 % et diluée dans une solution de cation monovalent

Une suspension virale est constituée à partir d'un adénovirus recombinant dans le génome duquel est inséré le gène humain codant pour la protéine CFTR (Ad-CFTR) tel que décrit dans Rosenfeld et al. (1992, supra).

Brièvement, il dérive de l'adénovirus de type 5, dont le génome est dépourvu, d'une part du gène E1A codant pour une protéine transactivatrice essentielle à la réplication de l'adénovirus et, d'autre part, du gène E3 non-essentiel. Le gène CFTR est inséré à la place du gène E1A.

L'Ad-CFTR est propagé au moyen de la lignée 293 de cellules de rein embryonnaire humain (Graham et al., 1977, J. Gen. Virol., 36, 59-72) qui exprime la fonction E1 de façon constitutive. Cette lignée est disponible à l'ATCC (CRL 1573). Les cellules 293 sont cultivées selon les recommandations du fournisseur.

Les cellules en culture sont infectées avant confluence par un inoculum initial de virus Ad-CFTR et selon une multiplicité d'infection (m.o.i.) de 2 à 10. On les incube à 37°C, et dès que l'on observe des effets cytopathiques, normalement après 4 à 10 jours de culture, les virus sont récoltés selon le protocole suivant.

On centrifuge la suspension cellulaire à basse vitesse pendant 10 minutes. Le culot cellulaire est resuspendu dans environ 10 ml de milieu GMEM (Glasgow Modified Eagle Medium, Gibco BRL, Cergy-Pontoise) additionné de 2 % de sérum de veau foetal. Les virus sont séparés des cellules par plusieurs cycles de congélation/décongélation successifs dans un bain d'éthanol-glace carbonique/bain-marie à 37°C. A la suite du dernier cycle, on élimine les débris cellulaires par centrifugation à basse vitesse pendant 5 à 10 minutes.

On purifie les virus à partir du surnageant de centrifugation, par fractionnement sur un gradient de chlorure de césium à deux étages de densité 1,40 et 1,25 g/ml respectivement. On centrifuge à 100 000 g pendant quelques heures à température ambiante. Les virus apparaissent sous forme d'une bande blanche située à l'interface des deux étages et sont récupérés à l'aide d'une seringue. Ils sont soumis à une deuxième purification sur gradient de chlorure de césium auto-formé préparé à l'aide d'une solution à 1,33 g/ml. Les virus sont également récupérés par ponction du tube avec une seringue et 1/10 du volume en glycérol est ajouté.

On élimine le chlorure de césium par dialyse contre un tampon contenant du Tris-HCl 10 mM, pH 7,4, MgCl₂ 1 mM et glycérol 10 %.

Le titre de la suspension virale ainsi obtenue est précisément déterminé selon la méthode du titrage en agar ou à 30 h (Graham et Prevec, 1991, Methods in Molecular Biology, 7, 109-128, ed. E.J. Murray, The Human Press Inc. Clinton, NJ). La suspension est répartie dans des tubes (Nunc ou Nalgène) en aliquote de 100 µl, de manière à contenir chacun 5x10⁹ à 5x10¹⁰ upf. Ces tubes qui constituent le stock de la suspension virale, sont entreposés à -60°C jusqu'à leur utilisation.

Au moment de l'utilisation, on ajoute à 100 µl de la suspension d'Ad-CFTR (à une concentration de 10⁷, 10⁸ ou 10⁹ ufp) 1,5 ml d'une solution de chlorure de sodium à 9 g/l (Meram, Melun). Les contrôles effectués montrent que le titre de la suspension virale diluée ainsi obtenue est stable plusieurs heures à température ambiante.

Une étude de stabilité accélérée a également été entreprise à 37°C. Après dilution de la suspension virale à différentes concentrations (10⁹, 10⁷, 10⁵ ufp/ml) dans la solution de chlorure de sodium précédente, on prélève un échantillon de chaque dilution à intervalle régulier (t = Oh, 2h, 4h et 24h) et on titre l'infectivité virale par la technique agar ou 30h. On constate la stabilité à 37°C de la suspension virale diluée pendant plus de 3h en particulier à forte concentration (10⁹ et 10⁷ ufp/ml).

On génère un aérosol en plaçant la suspension virale diluée dans le réservoir d'un nébuliseur Optineb 709 (Air Liquide, Paris). Il s'agit d'un appareil à génération pneumatique fonctionnant sous haute pression et offrant le choix du gaz vecteur. Il est relié à une source d'air comprimé et on peut appliquer une pression en air médical variable. On effectue des essais d'aérosolisation afin d'évaluer les conditions optimales à appliquer aux patients atteints de mucoviscidose. En particulier, on étudie l'effet de la pression (de 1 à 3,5 bars) et du rythme respiratoire. Celui-ci définit un nombre de respirations par minute et peut être prédéterminé par un réglage de l'appareil. A cet égard, le réglage en position 7 correspond au rythme respiratoire d'un enfant alors que la position 9 correspond à celui d'un adulte.

L'aérosolisation est réalisée en 3 étapes (suspension virale diluée suivie de deux rinçages de la coupelle par la solution diluante de chlorure de sodium). L'Optineb est réglé en autodéclenchement et relié à un piège ou collecteur par exemple le collectron MD8 (Sartorius, Palaiseau, FRANCE) au bout duquel on adapte une membrane filtrante en gélatine permettant de récupérer les particules virales après la nébulisation. Celle-ci est ensuite dissoute à 37°C dans un tampon Tris-HCl 10 mM pH 7,4, MgCl₂ 1 mM et glycérol 10 % et on titre l'infectivité des virus récupérés par la technique 30h et agar. Cette mesure permet d'évaluer la quantité de virus actifs capables de pénétrer dans les voies respiratoires d'un patient. Dans les différentes conditions testées et de façon relativement répétée, les virus infectieux piégés représentent de 15 à 40 % du nombre initial de virus. Bien que des fluctuations soient observées, les meilleurs rendements de récupération sont obtenus à forte pression (3,5 bars) ceci dans les deux positions de réglage. A titre indicatif, le rendement de récupération de médicaments appliqués par aérosolisation est habituellement d'environ 10 %. Ces résultats permettent d'envisager l'utilisation de l'aérosolisation d'adénovirus recombinants Ad-CFTR pour le traitement des patients atteints de mucoviscidose.

### EXEMPLE 2 : Préparation d'un aérosol à partir d'une suspension virale conservée en présence de saccharose 1M et diluée dans une solution de cation monovalent

L'exemple 1 est répété avec la variante suivante :

Après récupération des virus après le deuxième gradient de chlorure de césium, ceux-ci sont dialysés contre un tampon Tris-HCl 10 mM, pH 7,4, MgCl₂ 1 mM et saccharose 1M.

Les essais d'aérosolisation sont effectués comme décrit dans l'exemple 1 à une pression de 3,5 bars et un réglage de l'Optineb en position 9. On obtient un rendement de récupération de particules virales actives de 27 %.

### EXEMPLE 3 : Préparation d'un aérosol à partir d'une suspension virale diluée dans une solution de glucose

L'exemple 1 est répété avec la variante suivante :

On constitue une suspension virale diluée en ajoutant 100 µl de la suspension virale, à 1,5 ml d'une solution de glucose à 50 g/l (Laboratoire Chaix du Marais Lavoisier, Paris).

Lorsque l'on soumet la suspension virale diluée à une étude de stabilité accélérée à 37°C dans les mêmes conditions que celles définies dans l'exemple 1 à la différence que la dilution est effectuée dans la solution de glucose, on constate que l'activité virale est stable pendant plus de 2 heures.

### EXEMPLE 4 : Préparation d'un aérosol par vibration ultrasonique

L'exemple 1 est répété avec la variante suivante :

On génère un aérosol en mettant en oeuvre un nébuliseur ultrasonique SAM LS (System Assistance Medical, Le Lédat, France), utilisé selon les recommandations du fournisseur. La suspension virale diluée obtenue selon l'exemple 1, est déposée dans la coupelle du nébuliseur et on la soumet à une vibration ultrasonique provoquée par un quartz vibrant à une fréquence de 2,4 MHz. Dans ces conditions, le rendement de récupération est de l'ordre de 10 %.

### EXEMPLE 5 : Essais précliniques chez les primates

Le but de ces essais est d'établir que l'aérosolisation des adénovirus recombinants permet le transfert *in vivo* du gène CFTR dans les cellules pulmonaires et d'évaluer la toxicité du traitement.

### A. Mode opératoire

L'expérimentation inclut 5 singes Rhésus (Macaca mulatta) (TNO Center for Animal Research, Rijswijk, Pays-Bas). Les animaux sélectionnés sont en bonne santé et pèsent de 5 à 10 kg. Ils ont été répartis en trois groupes :
- Le groupe 1 est constitué de 2 singes auxquels on administre une seule dose d'Ad-CFTR (à J = 0), une dose élevée (7,5 x 10⁹ ufp) pour le premier et une dose faible (1,5 x 10⁷ ufp) pour le second. Ils sont sacrifiés à J + 10.
- Le groupe 2 est constitué également de 2 singes qui reçoivent deux administrations à 10 jours d'intervalle (J = 0 et J + 10) de doses équivalentes d'Ad-CFTR (respectivement 7,5 x 10⁹ ufp et 1,5 x 10⁷ ufp). Ceux-ci sont sacrifiés à J + 13.
- Le groupe contrôle comprend un singe traité par un fluide contrôle (Tris-HCl 10 mM pH 7,4 MgCl₂ 1 mM et glycérol 10 %) selon le protocole appliqué au groupe 2 (2 administrations successives à J = 0 et J + 10 avant d'être sacrifié à J + 13).
100 µl de la solution d'Ad-CFTR ajustée aux concentrations indiquées ci-dessus (groupe 1 et 2) ou 100 µl de solution contrôle (groupe 3) sont ajoutés à 1,5 ml d'une solution diluante de chlorure de sodium à 9 g/l et placés dans la coupelle d'un appareil Optineb. La nébulisation est réalisée sur les singes anesthésiés par l'intermédiaire d'un tube introduit dans la trachée et relié au nébuliseur. Les conditions opératoires sont les suivantes : réglage en position 7, pression en air médical de 3,5 bars et nébulisation en deux étapes (administration de la solution virale diluée puis rinçage avec 800 µl de solution diluante).

### B. Analyse du pouvoir de dissémination des virus

Les selles de chacun des animaux sont recueillies régulièrement et mises en culture sur cellules permissives 293 ou HepG2 afin de rechercher la présence de particules virales recombinantes ou sauvages respectivement. Après environ 3 et 7 jours de culture, celles-ci sont détectées par immunofluorescence indirecte à l'aide d'un anticorps monoclonal spécifique par exemple un anticorps reconnaissant la protéine capsidaire de l'adénovirus 5 (Bio-Science 012070). Ces analyses se sont avérées négatives chez tous les singes de l'expérimentation indiquant que le risque de dissémination du virus dans l'environnement est faible voire même nul.

### C. Analyse du transfert du gène CFTR in vivo

La méthodologie mise e oeuvre est décrite dans Bout et al. (1994, Hum. Gene Therapy, *5*, 3-10). Brièvement, on prélève sur les animaux sacrifiés les principaux organes (foie, testicules... etc) ainsi que des échantillons de tissus répartis tout au long des voies respiratoires. Une fois l'ADN extrait, on recherche les séquences adénovirales par PCR à l'aide d'amorces spécifiques de l'Ad-CFTR situées à la jonction de la cassette d'expression du CFTR et du vecteur adénoviral, respectivement OTG3042 (SEQ ID NO: 1, hybridant à la séquence CFTR) et OTG4347 (SEQ ID NO: 2, reconnaissant la séquence de l'adénovirus de type 5). A l'issue de 30 cycles d'amplification, les produits PCR sont séparés par électrophorèse sur gel d'agarose et transférés sur une membrane Hybond N+ (Amersham) avant d'être hybridés à une sonde spécifique (SEQ ID NO: 3) marquée au ³²P par phosphorylation. On obtient un signal spécifique uniquement dans la trachée et les poumons des singes du groupe 1 et 2 traités avec une dose virale élevée (7,5 x 10⁹ ufp). Il est intéressant de noter que tous les autres organes testés sont négatifs.

Il est nécessaire d'augmenter la sensibilité de la technique pour détecter la présence des séquences adénovirales chez les singes ayant reçu la dose virale de 1,5 x 10⁷ ufp. Pour ce faire, après 30 premiers cycles de PCR, 30 cycles supplémentaires sont effectués en mettant en oeuvre OTG3042 et une amorce interne OTG4908 (SEQ ID NO: 4). Puis les produits PCR sont traités comme indiqué précedemment. Un signal est obtenu dans environ la moitié des échantillons prélevés des voies respiratoires. Il semble donc que l'administration des virus par aérosol dans les poumons soit dose-dépendante. Bien entendu, l'hybridation est négative pour le singe contrôle.

Dans leur ensemble, ces résultats indiquent que l'Ad-CFTR reste contenu dans l'organe ciblé, à savoir les voies respiratoires.

### D. Expression in vivo du gène CFTR

L'expression du gène CFTR humain est étudiée dans les échantillons prélevés des voies respiratoires par la technique de reverse-transcription d'ARN suivie d'un PCR (Shouldiner et al., 1993, in Methods in Molecular Biology, *15*, 169-176, ed : white, Human Press Inc, Totowa, NJ). Cette technique qui a l'avantage d'être hautement spécifique, va permettre de révéler la présence d'ARNm codant pour le CFTR humain. Les ARN sont isolés des échantillons par les techniques conventionnelles. 1 µg d'ARN total est reverse -transcrit en présence de la reverse-transcriptase du MoMLV (Moloney Murine Leukemia Virus) en utilisant une amorce possédant à son extrémité 5' une séquence unique de repérage (dite tag en anglais). Plus précisement, celle-ci (OTG5987 ; SEQ ID NO: 5) comprend 30 nucléotides de séquence de repérage et 17 nucléotides correspondant au signal de polyadenylation et permettant la réassociation aux ARNm CFTR issus de la transcription de l'Ad-CFTR. Le PCR est ensuite réalisé directement sur le mélange réactionnel précédent en mettant en oeuvre les amorces OTG5988 (SEQ ID NO: 6) et OTG5999 (SEQ ID NO: 7 correspondant à la séquence de repérage de OTG5987) pour les 30 premiers cycles d'amplification puis OTG5988 et OTG4741 (SEQ ID NO: 8) pour 30 cycles supplémentaires. Les produits d'amplifications sont analysés comme décrit ci-dessus.

Les ARNm CFTR humains sont mis en évidence dans les échantillons de lobes pulmonaires des singes auxquels on a administré une ou deux doses virales de 7,5 x 10⁹ ufp. En revanche, aucun signal n'est détecté dans les échantillons provenant du singe contrôle ni des singes ayant reçu la dose virale faible.

Ces résultats montrent que la nébulisation de doses adéquates d'Ad-CFTR résulte en une expression du gène CFTR fonctionnel dans les cellules pulmonaires et sont particulièrement encourageants en vue du traitement de la mucoviscidose par thérapie génique.

### E. Pathologie

D'une manière générale, la nébulisation d'Ad-CFTR n'est pas associée à une perte de poids, un comportement anormal des singes ni à des perturbations significatives des paramètres sériques hématologiques et biochimiques.

Afin d'évaluer plus précisément les fonctions pulmonaires, des analyses histopathologiques post-mortem sont effectuées sur des coupes de tissus prélevés à différents endroits des voies respiratoires, selon la méthodologie décrite dans Bout et al. (1994, *supra*). Ces coupes sont examinées en vue de rechercher des signes d'inflammation et des lésions de l'épithélium pulmonaire. Les résultats sont les suivants. Dans tous les singes, l'architecture pulmonaire est intacte. On observe des manifestations inflammatoires mineures et des métaplasies squameuses sans correlation avec l'administration d'Ad-CFTR puisque également présentes chez le singe témoin.

Cependant, des lésions supplémentaires de faible intensité (infiltrations lymphocytaires périvasculaires et péribronchiques et pneumonie interstitielle) ont été notées chez les 2 singes ayant reçu une dose virale élevée sans qu'aucune différence significative n'apparaisse entre les animaux du groupe I (nébulisation d'une dose de 7,5 x 10⁹ ufp) et ceux du groupe II (nébulisation de deux doses de 7,5 x 10⁹ ufp).

En conclusion, l'administration par aérosol d'Ad-CFTR est bien tolérée par l'ensemble des animaux traités. Ces études ont permis de s'assurer que le virus recombinant Ad-CFTR reste contenu dans le poumon et ne se dissémine pas dans l'organisme. Bien que des lésions pulmonaires soient observées suite à l'administration de doses virales élevées, il ne semble pas qu'elles conduisent à des pathologies graves. Ces essais précliniques permettent de conclure à la faisabilité du traitement chez l'homme.

## Revendications

1. Procédé d'obtention d'un aérosol viral, caractérisé en ce que l'on prépare une suspension virale diluée correspondant à la dilution d'une suspension virale contenant de 10⁴ à 10¹³ ufp de virus dans une solution aqueuse contenant au moins de 6 à 12 g/l d'un sel d'un cation monovalent ou de 50 à 100 g/l d'un hexose.

2. Procédé selon la revendication 1, caractérisé en ce que la suspension virale est une suspension d'un virus associé aux adénovirus ou d'un adénovirus recombinant.

3. Procédé selon l'une des revendications 1 et 2, caractérisé en ce que ladite dilution est effectuée selon un rapport en volume suspension virale/solution aqueuse de 1/5 à 1/20.

4. Procédé selon la revendication 3, caractérisé en ce que ladite dilution est effectuée selon un rapport en volume suspension virale/solution aqueuse de 1/10 à 1/20.

5. Procédé selon la revendication 3, caractérisé en ce que ladite dilution est effectuée selon un rapport en volume suspension virale/solution aqueuse de 1/12 à 1/18.

6. Procédé selon une des revendications 1 à 5, caractérisé en ce qu'on dilue une suspension virale dans une solution aqueuse comprenant de 6 à 12 g/l d'un sel d'un cation monovalent sélectionné parmi le chlorure de sodium et le chlorure de potassium.

7. Procédé selon la revendication 6, caractérisé en ce qu'on dilue une suspension virale dans une solution aqueuse comprenant environ 9 g/l d'un sel d'un cation monovalent.

8. Procédé selon l'une des revendications 1 à 7, caractérisé en ce qu'on dilue une suspension virale dans une solution aqueuse comprenant de 50 à 100 g/l d'un hexose sélectionné parmi le glucose et le mannose.

9. Procédé selon la revendication 8, caractérisé en ce qu'on dilue une suspension virale dans une solution aqueuse comprenant 50 g/l de glucose.

10. Procédé selon l'une des revendications 1 à 9, caractérisé en ce que la suspension virale est une suspension d'un virus recombinant dans le génome duquel est inséré un fragment d'ADN codant pour une protéine hétérologue à ce virus.

11. Procédé selon la revendication 10, caractérisé en ce que la suspension virale est une suspension d'un virus recombinant dans le génome duquel est inséré un fragment d'ADN codant pour la protéine CFTR.

12. Procédé selon l'une des revendications 1 à 11, caractérisé en ce que la suspension virale comprend de 10⁶ à 10¹² ufp de virus.

13. Procédé selon la revendication 12, caractérisé en ce que la suspension virale comprend de 10⁸ à 10¹¹ ufp de virus.

14. Utilisation d'un aérosol viral susceptible d'être obtenu par le procédé selon l'une des revendications 1 à 13 pour la préparation d'un médicament administré par nébulisation de ladite suspension virale diluée avec une pression de gaz de 0,5 à 3,5 bars ou une fréquence ultrasonore de 2 à 5 MHz.

15. Utilisation d'un aérosol viral selon la revendication 14, caractérisée en ce que la pression de gaz est comprise entre 2 et 3,5 bars.

16. Utilisation d'un aérosol viral selon la revendication 14, caractérisée en ce que la fréquence ultrasonore est comprise entre 2 et 3 MHz.

17. Utilisation d'un aérosol viral selon l'une des revendications 14 à 16, pour la préparation d'un médicament destiné au traitement d'une maladie pulmonaire.

18. Utilisation selon la revendication 17, pour la préparation d'un médicament destiné au traitement de la mucoviscidose, de l'emphysème pulmonaire, de l'asthme ou du cancer des poumons.

19. Aérosol viral susceptible d'être obtenu par la mise en oeuvre du procédé selon l'une des revendications 1 à 13.

## Patentansprüche

1. Verfahren zur Herstellung eines viralen Aerosols, dadurch charakterisiert, daß man eine verdünnte virale Suspension herstellt, die der Verdünnung einer viralen Suspension entspricht, welche 10⁴ bis 10¹³ ufp (plaquebildende Einheiten) des Virus in einer wäßrigen Lösung enthält, enthaltend mindestens 6 bis 12 g/l eines Salzes eines monovalenten Kations oder 50 bis 100 g/l einer Hexose.

2. Verfahren nach Anspruch 1, dadurch charakterisiert, daß die virale Suspension eine Suspension eines Virus ist, der mit Adenoviren zusammenhängt, oder eines rekombinanten Adenovirus.

3. Verfahren nach einem der Ansprüche 1 und 2, dadurch charakterisiert, daß die Verdünnung nach einem Volumenverhältnis virale Suspension/wäßrige Lösung von 1/5 bis 1/20 durchgeführt wird.

4. Verfahren nach Anspruch 3, dadurch charakterisiert, daß die Verdünnung nach einem Volumenverhältnis virale Suspension/wäßrige Lösung von 1/10 bis 1/20 durchgeführt wird.

5. Verfahren nach Anspruch 3, dadurch charakterisiert, daß die Verdünnung nach einem Volumenverhältnis virale Suspension/wäßrige Lösung von 1/12 bis 1/18 durchgeführt wird.

6. Verfahren nach einem der Ansprüche 1 bis 5, dadurch charakterisiert, daß man eine virale Suspension in einer wäßrigen Lösung verdünnt, die 6 bis 12 g/l eines Salzes eines monovalenten Kations umfaßt, welches ausgewählt wird aus Natriumchlorid und Kaliumchlorid.

7. Verfahren nach Anspruch 6, dadurch charakterisiert, daß man eine virale Suspension in einer wäßrigen Lösung verdünnt, die ungefähr 9 g/l eines Salzes eines monovalenten Kations umfaßt.

8. Verfahren nach einem der Ansprüche 1 bis 7, dadurch charakterisiert, daß man eine virale Suspension in einer wäßrigen Lösung verdünnt, die 50 bis 100 g/l einer Hexose umfaßt, die aus Glucose und Mannose ausgewählt wird.

9. Verfahren nach Anspruch 8, dadurch charakterisiert, daß man eine virale Suspension in einer wäßrigen Lösung verdünnt, die 50 g/l Glucose umfaßt.

10. Verfahren nach einem der Ansprüche 1 bis 9, dadurch charakterisiert, daß die virale Suspension eine Suspension eines rekombinanten Virus ist, in dessen Genom ein DNA-Fragment insertiert ist, das für ein heterologes Protein dieses Virus kodiert.

11. Verfahren nach Anspruch 10, dadurch charakterisiert, daß die virale Suspension eine Suspension eines rekombinanten Virus ist, in dessen Genom ein DNA-Fragment insertiert ist, das für das Protein CFTR kodiert.

12. Verfahren nach einem der Ansprüche 1 bis 11, dadurch charakterisiert, daß die virale Suspension 10⁶ bis 10¹² ufp des Virus umfaßt.

13. Verfahren nach Anspruch 12, dadurch charakterisiert, daß die virale Suspension 10⁸ bis 10¹¹ ufp des Virus umfaßt.

14. Verwendung eines viralen Aerosols, erhältlich nach dem Verfahren nach einem der Ansprüche 1 bis 13, zur Herstellung eines Medikaments, das durch Vernebelung dieser verdünnten viralen Suspension mit einem Gasdruck von 0,5 bis 3,5 bar oder einer Ultraschall-Frequenz von 2 bis 5 MHz gegeben wird.

15. Verwendung eines viralen Aerosols nach Anspruch 14, dadurch charakterisiert, daß der Gasdruck zwischen 2 und 3,5 bar liegt.

16. Verwendung eines viralen Aerosols nach Anspruch 14, dadurch charakterisiert, daß die Ultraschall-Frequenz zwischen 2 und 3 MHz liegt.

17. Verwendung eines viralen Aerosols nach einem der Ansprüche 14 bis 16 zur Herstellung eines Medikaments, das zur Behandlung einer Lungenkrankheit bestimmt ist.

18. Verwendung nach Anspruch 17 zur Herstellung eines Medikaments, das zur Behandlung von Mucoviscidose, Lungenemphysem, Asthma oder Lungenkrebs bestimmt ist.

19. Virales Aerosol, erhältlich durch Durchführung des Verfahrens nach einem der Ansprüche 1 bis 13.

## Claims

1. Method for obtaining a viral aerosol, characterized in that a dilute viral suspension is prepared corresponding to the dilution of a viral suspension containing 10⁴ to 10¹³ pfu of virus in an aqueous solution containing at least 6 to 12 g/l of a salt of a monovalent cation or 50 to 100 g/l of a hexose.

2. Method according to Claim 1, characterized in that the viral suspension is a suspension of an adeno-associated virus or of a recombinant adenovirus.

3. Method according to either of Claims 1 and 2, characterized in that the said dilution is performed according to a viral suspension/aqueous solution ratio by volume of 1:5 to 1:20.

4. Method according to Claim 3, characterized in that the said dilution is performed according to a viral suspension/aqueous solution ratio by volume of 1:10 to 1:20.

5. Method according to Claim 3, characterized in that the said dilution is performed according to a viral suspension/aqueous solution ratio by volume of 1:12 to 1:18.

6. Method according to one of Claims 1 to 5, characterized in that a viral suspension is diluted in an aqueous solution comprising 6 to 12 g/l of a salt of a monovalent cation selected from sodium chloride and potassium chloride.

7. Method according to Claim 6, characterized in that a viral suspension is diluted in an aqueous solution comprising approximately 9 g/l of a salt of a monovalent cation.

8. Method according to one of Claims 1 to 7, characterized in that a viral suspension is diluted in an aqueous solution comprising 50 to 100 g/l of a hexose selected from glucose and mannose.

9. Method according to Claim 8, characterized in that a viral suspension is diluted in an aqueous solution comprising 50 g/l of glucose.

10. Method according to one of Claims 1 to 9, characterized in that the viral suspension is a suspension of a recombinant virus into the genome of which a DNA fragment coding for a protein heterologous to this virus is inserted.

11. Method according to Claim 10, characterized in that the viral suspension is a suspension of a recombinant virus into the genome of which a DNA fragment coding for the CFTR protein is inserted.

12. Method according to one of Claims 1 to 11, characterized in that the viral suspension comprises 10⁶ to 10¹² pfu of virus.

13. Method according to Claim 12, characterized in that the viral suspension comprises 10⁸ to 10¹¹ pfu of virus.

14. Use of a viral aerosol which can be obtained by the process according to one of Claims 1 to 13, for the preparation of a medicinal product administered by nebulizing the said dilute viral suspension with a gas pressure of 0.5 to 3.5 bar or an ultrasound frequency of 2 to 5 MHz.

15. Use of a viral aerosol according to Claim 14, characterized in that the gas pressure is between 2 and 3.5 bar.

16. Use of a viral aerosol according to Claim 14, characterized in that the ultrasound frequency is between 2 and 3 MHz.

17. Use of a viral aerosol according to one of Claims 14 to 16, for the preparation of a medicinal product intended for the treatment of a lung disease.

18. Use according to Claim 17, for the preparation of a medicinal product intended for the treatment of cystic fibrosis, pulmonary emphysema, asthma or lung cancer.

19. Viral aerosol which can be obtained by implementing the method according to one of Claims 1 to 13.


### LISTE DE SEQUENCES

(1) INFORMATION GENERALE:
   (i) DEPOSANT:
      (A) NOM: Transgene S.A.
      (B) RUE: 11 rue de Molsheim
      (C) VILLE: Strasbourg
      (E) PAYS: France
      (F) CODE POSTAL: 67082
      (G) TELEPHONE: (33) 88 27 91 00
      (H) TELECOPIE: (33) 88 22 58 07
   (ii) TITRE DE L' INVENTION: Procede de preparation d'un aerosol viral.
   (iii) NOMBRE DE SEQUENCES: 8
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 23 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: ADNc CFTR humain
      (C) INDIVIDUEL ISOLE: oligonucleotide synthetique
   (vii) SOURCE IMMEDIATE:
      (B) CLONE: OTG3042
   (xi) DESCRIPTION DE LA SEQUENCE: SEQ ID NO: 1:
   GCAGTTGATG TGCTTGGCTA GAT 23
(2) INFORMATION POUR LA SEQ ID NO: 2:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Adenovirus de type 5
      (C) INDIVIDUEL ISOLE: oligonucleotide de synthese
   (vii) SOURCE IMMEDIATE:
      (B) CLONE: OTG4347
   (xi) DESCRIPTION DE LA SEQUENCE: SEQ ID NO: 2:
   CAGCTCCTCG GTCACATCCA G 21
(2) INFORMATION POUR LA SEQ ID NO: 3:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: ADNc CFTR humain
      (C) INDIVIDUEL ISOLE: oligonucleotide de synthese
   (vii) SOURCE IMMEDIATE:
      (B) CLONE: OTG4905
   (xi) DESCRIPTION DE LA SEQUENCE: SEQ ID NO: 3:
   TGCTGCTCTC TAAAGCCTTG TATC 24
(2) INFORMATION POUR LA SEQ ID NO: 4:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Adenovirus de type 5
      (C) INDIVIDUEL ISOLE: oligonucleotide de synthese
   (vii) SOURCE IMMEDIATE:
      (B) CLONE: OTG4908
   (xi) DESCRIPTION DE LA SEQUENCE: SEQ ID NO: 4:
   TCGTACCTCA GCACCTTCCA GATC 24
(2) INFORMATION POUR LA SEQ ID NO: 5:
   (i) CARACTERISTIQUES DE LA SEQUENCE:
      (A) LONGUEUR: 47 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (iii) HYPOTHETIQUE: NON
   (iii) ANTI-SENS: OUI
   (vi) ORIGINE:
      (C) INDIVIDUEL ISOLE: oligonucleotide de synthese
   (vii) SOURCE IMMEDIATE:
      (B) CLONE: OTG5987
   (xi) DESCRIPTION DE LA SEQUENCE: SEQ ID NO: 5:
   CTTATACGGA TATCCTGGCA ATTCGGACTT ATTTGTGATG CTATTGC 47
(2) INFORMATION POUR LA SEQ ID NO: 6:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: ADNc CFTR humain
      (C) INDIVIDUEL ISOLE: oligonucleotide de synthese
   (vii) SOURCE IMMEDIATE:
      (B) CLONE: OTG5988
   (xi) DESCRIPTION DE LA SEQUENCE: SEQ ID NO: 6:
   AAGCAGTTGA TGTGCTTGGC TAGATCTGTT 30
(2) INFORMATION POUR LA SEQ ID NO: 7:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (C) INDIVIDUEL ISOLE: oligonucleotide de synthese
   (vii) SOURCE IMMEDIATE:
      (B) CLONE: OTG5999
   (xi) DESCRIPTION DE LA SEQUENCE: SEQ ID NO: 7:
   CTTATACGGA TATCCTGGCA ATTCGGACTT                                30
(2) INFORMATION POUR LA SEQ ID NO: 8:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Rhesus macaque polyoma virus (SV40)
      (C) INDIVIDUEL ISOLE: oligonucleotide de synthese
   (vii) SOURCE IMMEDIATE:
      (B) CLONE: OTG4741
   (xi) DESCRIPTION DE LA SEQUENCE: SEQ ID NO: 8:
   GTAACCATTA TAAGCTGCAA TAAAC                                     25